# EUROPEAN PATENT SPECIFICATION

(11) **EP 4 359 037 B1**
(45) Date of publication and mention of the grant of the patent: **28.10.2026**
(21) Application number: 22728347.0
(22) Date of filing: 03.05.2022
(51) Int. Cl.: A61M 5/24, A61M 5/20, A61M 5/315, F16H 1/32

(54) **DRIVE FOR DRUG DELIVERY DEVICE**
ANTRIEB FÜR EINE VORRICHTUNG ZUR ARZNEIMITTELABGABE
ENTRAÎNEMENT POUR DISPOSITIF D'ADMINISTRATION DE MÉDICAMENTS

(30) Priority: 24.06.2021 EP 21181373
(43) Date of publication of application: 01.05.2024
(73) Proprietor: Ypsomed AG, 3401 Burgdorf (CH)
(72) Inventor: HOSTETTLER, Patrick, 3415 Hasle (CH); BINGGELI, Nicolas, 3400 Burgdorf (CH)
(74) Representative: Kleiner, Stefan
(86) International application number: PCT/EP2022/061865
(87) International publication number: WO 2022/268389

(56) References cited:
- EP-A1- 0 020 314
- US-A1- 2007 225 657
- US-A1- 2015 216 681
- US-A1- 2021 023 307

## Description

### Technical Field

The invention relates to a drive for a drug delivery device, preferably for an automatic injection device. Further, the invention also relates to a drug delivery device with a drive.

### Background Art

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens.

An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the drug delivery device. A dose is dispensed by inserting the needle into a suited portion of human skin and by moving the piston manually or by pressing a release button of an automatic injection device.

There are several types of injection devices known in the art. Disposable injection devices are adapted to deliver the medicament from a container such as a pre-filled cartridge or syringe that is not intended to be replaced or refilled by the patient. Reusable, semi-disposable, or hybrid delivery devices have a components of the device that may be replaced by the patient, or a cartridge that may be refilled, while some components of the device may be reused with the replaced or refilled drug container.

Automatic injection devices with an electric motor usually require an adequate gear such as to reduce the relatively high rotation speed of the electric motor into a relatively slow linear movement of a piston arranged within the container barrel. Such a gear needs to be able to transmit a high torque, as the advancement of the piston requires some force, as it is used to eject the medicament from the container barrel through a needle or cannula into a patient. Further, the gear arrangement should have small dimensions so that the overall size of the injection device may be kept small.

The prior art discloses the use of a cycloidal drive in drug delivery devices as being suitable to fulfil the criteria mentioned above, as cycloidal drives are compact and provide a high gear ratio. For example, US 2007/0225657 proposes to use a cycloidal drive to transmit a rotational movement between a dose setting mechanism and a rotating scale. Further, WO 2014/117944 describes the use of a cycloidal drive in a dose setting mechanism.

US 2021/0023307 A1 discloses a reusable autoinjector comprising a linear actuator that drives a lead screw to dispensing medication from a dose cassette. The actuator includes an electric motor which rotates the lead screw in dispensing direction via gearbox.

US 2007/0225657 A1 discloses an injection device with a dose setting mechanism comprising a cycloid gear. An eccentric sleeve rotates eccentrically about a longitudinal axis of the injection device and an outer circumference of the eccentric sleeve engages an inner circumference of a cycloid disc. The cycloid gear provides a gear ratio such that the cycloid disc rotates significantly slower than the eccentric sleeve.

EP 0020314 A1 relates to the fundamental function of a cycloidal transmission. The document discloses a drive with an eccentric roller that supports a roller carrier which at its outer periphery has several grooves for a set of rollers. The rollers cooperate with a cycloidal cam curve disc which is secured to a housing and which is formed with several cam curves and lobe curves which are adapted to the rollers. A cam disc carrier partly encloses the roller carrier and includes a second cycloidal cam curve disc. The second cam curve disc is designed to match the rollers and the eccentricity of the eccentric roller so that the rollers roll over the cam surfaces and the lobe surfaces of the cam curve disc when the roller carrier rotates.

### SUMMARY OF THE INVENTION

It is the object of the invention to create a drive for a drug delivery device which is small in dimension, provides a high gear ratio and which allows the transmission of high torque.

The solution of the invention is specified by the features of claim 1. According to the invention, the drive for a drug delivery device comprises a drive mechanism with a drive shaft, a pinion arranged on the drive shaft and a cycloidal drive. The cycloidal drive has a cycloidal disc and a ring gear, preferably a stationary ring gear. The cycloidal disc is driven in an eccentric motion by means of a planet gear which meshes with the pinion on the drive shaft and with an internal gear of the cycloidal disc.

The inventive combination of a cycloidal drive with a planetary drive allows to increase the gear ratio of the cycloidal drive without increasing the size of the gear arrangement, as the planetary drive is arranged inside the cycloidal drive. Further, as planetary gears are suitable to transmit high torques, the torque transmission of the total gear arrangement is not negatively affected. A further big advantage of the drive according to the present invention is that it uses a very low number of different parts, i.e. only four parts beside the drive mechanism. Hence, this drive is very easy to assemble and may be manufactured at low costs.

The drive mechanism may include a manual drive mechanism, an electric drive such as an electric motor or a mechanical drive such as mechanical spring or elastic element adapted to be biased and released.

The electric motor may be of any suitable type, such as a brushed or brushless DC motor. Preferably, the electric motor comprises a closed-loop control system allowing a precise control of the electric motor, such as for example a servomotor. Of course, the electric motor should have a small enough dimension in order to fit into a housing of a drug delivery device in which the drive is to be used.

The pinion is preferably permanently attached to the drive shaft of the drive mechanism, e.g. by means of a press fit, an adhesive or any other suitable means. Alternatively, the pinion may be integral with the drive shaft, the pinion may for example be milled on the drive shaft.

The cycloidal drive uses preferably a stationary or a rotationary ring gear as well as a cycloidal disc in the known matter. In a preferred embodiment the ring gear comprises a plurality of pins on an inner surface thereof which protrude in a radial direction towards the center of the ring gear. Alternatively, the ring gear may include a rotational wheel or sleeve compensating the eccentric movement of the cycloidal disc by means of an auxiliary gear, wheels or rollers. The cycloidal disc eccentrically moves around the inner surface of the ring gear, i.e. the cycloidal disc is moved in a wobbling motion around the inner surface of the ring gear. Preferably, the cycloidal disc comprises a number of straight teeth which protrude radially outward and which preferably interact with the pins of the ring gear. The number of straight teeth of the cycloidal ring is smaller than the number of pins of the ring gear. Hence, the eccentric movement of the cycloidal disc within the ring gear causes the cycloidal disc to rotate. The cycloidal disc is shaped as a ring which preferably comprises the aforementioned straight teeth on an outer surface as well as an internal gear with gear teeth on an inner surface.

The term gear is not limited to a pin or teeth mechanism but may instead include wheels with a flat surface without pins or teeth. In this case the disc and the ring gear may engage to each other by friction only.

The cycloidal disc is driven in the eccentric movement by means of the one planet gear which is arranged between the cycloidal disc and the pinion. With this arrangement, a rotational movement of the drive shaft and the pinion in a first direction is translated into a rotational movement of the planet gear into a second rotational movement, which is opposite to the first rotational movement. It is to be noted that the planet gear itself moves around the pinion in the first direction as it meshes with the internal gear of the cycloidal ring. Hence, the planet gear undergoes two kinds of movements at the same time: First, it rotates around its central axis in the second direction and second it moves around the pinion in the second direction. This joint movement of the planet gear drives the cycloidal disc into the aforementioned eccentric movement.

Examples for a drug delivery device may be a disposable injection pen comprising a cartridge with the liquid drug, a semi-disposable injection pen with a reusable drive unit and a replaceable receptacle including a cartridge, a reusable injection pen with replaceable cartridge or an autoinjector comprising a syringe (in particular a semi-disposable autoinjector with a reusable drive unit and a replaceable receptacle including a syringe).

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

Preferably, the drive mechanism is an electric motor and preferably a stepper motor. Use of a stepper motor allows a precise control of the drive, without the need of control electronics or of a closed control loop. This enables to use the drive for delivering a controlled dose of a medicament by means of the drug delivery device the drive is being used in. Further, stepper motors are readily available and cheap, so that the costs for the drive may be considerably reduced.

Alternatively, the electric motor is a servomotor or a synchronous motor.

In a preferred embodiment the drug delivery device is an electronic injection device including the drive mechanism with the electric motor. In this case the motor is used to drive the drive shaft to dispense the drug. The electronic injection pen is preferably pen-shaped. The electronic injection device preferably comprises a drive member or a clutch sleeve, wherein the cycloidal disc is rotationally coupled to the drive member or clutch sleeve such that a rotational movement of the cycloidal disc is transferred to the drive member or clutch sleeve.

Preferably, the pinion, the cycloidal disc, the ring gear and/or the planet gear are made of a polymer material. Use of a polymer material for these components allows their manufacture by means of injection molding. Hence, large numbers may be manufactured in a quick and cheap manner.

The polymer material preferably is a durable thermoplastic, such as polycarbonate (PC), polystyrene (PS), acrylonitrile butadiene styrene (ABS), poly(methyl methacrylate) (PMMA), polyoxymethylene (POM), polyethylene terephthalate (PET), polyphenylene sulfide (PPS), polyimide (PI), polyetherimide (PEI), polysulfone (PS), polyvinylidene chloride (PVDC), polyether ether ketone (PEEK), polyphenylene sulfide (PPS), polytetrafluoroethylene (PTFE), polyvinylidene fluoride (PVDF) or polyamide (PA). Preferably, the pinion, the cycloidal disc, the ring gear and/or the planet gear are all made of the same polymer. However, alternatively, the pinion, the cycloidal disc, the ring gear and/or the planet gear may be made of different polymers.

Preferably, the polymer material is a medical grade polymer material, i.e. polymer material which fulfils the requirement of ISO 10993 and/or of the EU regulation 2017/745.

Preferably, the planet gear has helical teeth (inclined teeth) arranged on a peripheral surface. Concurrently, the pinion as well as the internal gear of the cycloidal disc have matching helical teeth. The use of helical teeth has the advantage to provide a smooth and quiet run of the gear components.

Alternatively the planet gear may comprise a straight toothing.

Preferably, the planet gear and the cycloidal drive are configured to provide a reduction gear ratio of at least 50:1, preferably of at least 100:1 between the drive shaft and the cycloidal disc. This means that the cycloidal disc turns around a full rotation for every at least 50, preferably at least 100 full rotations of the pinion and the drive shaft.

Preferably, the ring gear has an outer diameter which corresponds to or which is smaller than an outer diameter of a housing of the drive mechanism, preferably of an electric motor. Hence, the drive mechanism is the largest component of the whole drive, i.e. the drive according to the present invention will fit into any drug delivery device which has a sufficient diameter to receive the drive mechanism.

Preferably, the drive mechanism has a diameter of 10 mm or less. Hence it is possible to use the drive for small and handy drug delivery devices.

The cycloidal disc preferably comprises a cylindrical protrusion which extends from the cycloidal disc in an axial direction which faces away of the drive mechanism. This cylindrical protrusion may be used to connect the drive to further components of a drug delivery device in which the drive is used in. The protrusion preferably has a diameter which is equal to or smaller than the diameter of the cycloidal disc.

Preferably, the ring gear of the drive is a rotational ring gear and includes an outer rotation sleeve and at least three auxiliary wheels or rollers circumferentially arranged on an inside of the rotation sleeve. The auxiliary wheels or rollers mesh with an external gear of the cycloidal disc and thus are radially arranged between the cycloidal disc and the outer rotation sleeve.

That means the cycloidal disc rotates by its eccentric movement the auxiliary rollers as the rollers follow an outer surface of the external gear (e. g. teeth) of the cycloidal disc. The rotating rollers in turn rotate the outer rotation sleeve. The latter may be coupled to a clutch or drive member to drive a piston rod of the drug delivery device.

Hence, the auxiliary rollers and the outer rotational sleeve provide a transformation of the eccentric movement of the cycloidal disc in a pure rotational movement around a central or longitudinal axis of the drive and of the delivery device.

The outer rotational sleeve may be rotationally coupled to, for example, a drive member or a clutch sleeve adapted to drive the piston rod in a dispensing direction. Alternatively, the outer rotational sleeve is an integral part of the drive member or clutch sleeve.

The present application also relates to a drug delivery device including a longitudinal housing and a plunger rod, the plunger rod being adapted to advance a piston inside a reservoir containing a medicament in order to dispense the medicament. The plunger rod is driven in a dispensing direction by means of the drive according to the present invention in order to advance the piston.

The drug delivery device preferably is an injection device. An injection device is removed from an injection site after each administration of a dose of a medicament. Alternatively, the drug delivery device may be an infusion device, i.e. a device with a cannula or needle which remains in the skin of a patient for a period of time, e.g. several hours or days in order to administer several doses of the medicament to the patient during the period of time. More preferably, however, the drug delivery device is a pen-shaped injection device.

The housing preferably is elongated and has a round, oval, rectangular or polygonal cross-section. The cross section may vary in size and form along the central axis of the housing. Preferably, the housing is made of a polymer material, more preferably of a medical grade polymer material, i.e. polymer material which fulfil the requirement of ISO 10993 and/or of the EU regulation 2017/745. The housing has a distal end and a proximal end. The distal end is the end of the housing which points towards a cannula or needle used to administer the medicament to a patient and which is connected to the drug delivery device. In the case that the drug delivery device is an injection device, the distal end of the housing will thus point towards the skin of a patient during an injection event. The proximal end of the housing therefore is the end of the housing which faces away of the needle or cannula. In the case that the drug delivery device is an injection device, the proximal end of the housing faces away of the skin of the patient during an injection event.

The housing is preferably open at its proximal and distal end such as to allow the assembly of the drug delivery device. The proximal and distal ends are closed with appropriate lids or the like in order to protect the elements of the drug delivery device located within the housing.

The drug delivery device preferably comprises a reservoir with a medicament. The reservoir may be located within the housing in certain embodiments. In this case, the drug delivery device is preferably discarded after all of the medicament in the reservoir has been administered to the patient. Preferably, however, the reservoir is included in a cartridge which is removably connectable with the housing, preferably at the distal end of the housing.

The reservoir comprises a piston which is movable in order to dispense the medicament through a cannula or needle. The cannula or needle is preferably connected or connectable to the distal end of the housing, of the cartridge or of the reservoir.

The medicament preferably is a medicament used to treat diabetes. Preferably, the medicament is insulin or a derivative thereof.

The plunger rod is moved linearly about the central axis by the drive. By means of movement of the plunger rod, the piston is moved within the reservoir in order to dispense the medicament. Hence, the drug delivery device is an automatic drug delivery device where the medicament is dispensed upon actuation of a button or the like by the patient. The plunger rod is preferably arranged such that its length axis is congruent with a central axis of the housing.

As the output of the drive is a rotational movement, the drug delivery device comprises means to transform the rotational movement of the drive into the linear movement of the plunger rod. In certain preferred embodiments, the plunger rod may additionally be driven in rotation in addition to the linear movement. Such a rotational movement of the plunger rod may of course be used to drive the plunger rod in the linear direction.

The drug delivery device preferably comprises a dispensing button which may be depressed by a patient in order to dispense a dose of medicament with the drug delivery device. Preferably, the drug delivery device further comprises a control element which is connected with the drive mechanism as well as with the dispensing button in order to switch on the drive mechanism for a defined period of time when the dispensing button is depressed.

The drug delivery device preferably comprises a dose setting arrangement which allows the setting of the dose of medicament which is to be administered with the drug delivery device. The dose setting arrangement preferably comprises a dose setting wheel which may be rotated by the patient in order to adjust the dose and an indicator, preferably a screen, which shows the set dose to the patient. The set dose is delivered by activating the device for a defined time, which is calculated in function of parameters of the volume and/or diameter of the reservoir as well as the linear speed of the plunger rod.

Further, the drug delivery device preferably comprises a dose counter which indicates the amount of medicament which has been dispensed from or which remains in the reservoir.

The housing comprises a source of electrical energy, such as a primary battery, a secondary battery or a supercapacitor in order to provide power to a possibly present electric drive mechanism.

Preferably, the cycloidal disc is rotationally coupled to a drive member which is configured as hollow cylinder being concentrically arranged around the plunger rod. The plunger rod has a thread on an outer circumference which meshes with the drive member in a distal area thereof. The plunger rod further comprises at least one spline which is engaged with at least one locking pin such as to prevent the plunger rod from rotating.

This configuration provides a simple and reliable means of transforming the rotational movement of the drive into a linear movement of the plunger rod. The drive member itself is not moved linearly about the housing and hence the connection between the cycloidal disc of the drive and the drive member may be kept simple. For example, the drive member may be connected to the cycloidal disc or a protrusion extending therefrom by means of a form fit connection. Preferably, the connection between the cycloidal disc and the drive member is realized by means of teeth on an inner surface of a proximal area of the drive member meshing with matching teeth located on an outside surface of the protrusion extending from the cycloidal disc. As the cycloidal disc is driven in an eccentric rotation, the protrusion is likewise moving eccentrically. The diameter of the inner surface of the proximal area of the drive member is hence preferably chosen such that it equals the diameter of the outermost rotational path of the outer surface of the protrusion. Hence, the protrusion may eccentrically rotate within the proximal area of the drive member while nonetheless always being in contact therewith at one location where the teeth may mesh with each other. Hence, the eccentric rotation of the protrusion and the cycloidal disc may be transformed in a centric rotation of the drive member by simple but reliable means.

The plunger rod is prevented from rotating by means of the engagement of the at least one locking pin with the at least one spline of the plunger rod. As the plunger rod is in meshing engagement with the distal area of the drive member, the rotation of the drive member is transformed into a linear movement of the plunger rod when the drive member is driven in rotation.

Preferably, the drive member is arranged within a holder which is in the form of a hollow cylinder.

Alternatively, in a plunger rod rotating configuration the plunger rod is in threaded engagement with a housing of the delivery device and the plunger rod is rotationally splined and fixed to the drive member. Hence, the plunger rod screws in a distal and dispensing direction during dose delivery.

That means the cycloidal disc is preferably rotationally coupled to a drive member which is configured as hollow cylinder being concentrically arranged around the plunger rod, wherein the plunger rod is rotationally coupled to the drive member, wherein the plunger rod has a thread on an outer circumference which meshes with the housing or an insert of the housing of the drug delivery device.

The drug delivery device preferably includes a drive member wherein a proximal portion of the drive member is rotationally coupled to the cycloidal disc or forms the cyloidal disc and wherein the proximal portion is free to move eccentrically with the cycloidal disc and wherein a distal portion of the drive member is rotationally guided.

This allows an easy design of the mechanism as the proximal portion of the drive member does not have to be supported or guided and can move along with the the cycloidal disc. In contrast, the distal portion of the drive member is rotationally guided, preferably by a housing of the delivery device and hence the distal portion is restricted to rotational movement.

The drug delivery device comprises preferably a reset mechanism including a coupling member adapted to selectively couple the plunger rod to the drive member such that a dispensing movement of the drive member is transferred to the plunger rod and to decouple the plunger rod from the drive member such that a movement of the drive member is not transferred to the plunger rod and vice versa.

This provides a reset function as the plunger rod can be pulled back into the housing if the plunger rod is decoupled from the drive member, for example, during exchange of an empty cartridge or reservoir of the drug delivery device.

The coupling member can be an interlocking member such as a teeth connection or a splined engagement that are releasable to decouple the plunger rod from the drive member.

Other advantageous embodiments and combinations of features may be derived from the detailed description below and the entirety of the claims.

### Brief description of the drawings

The drawings used to explain the embodiments show:
- Fig. 1: an exploded view of an inventive drive for a drug delivery device;
- Fig. 2: a perspective view of the drive according to Fig. 1 in an assembled state;
- Fig. 3: the drive according to Fig. 1 in a plain view onto a face thereof;
- Fig. 4: a perspective length section showing the interaction of the drive as shown in Fig. 1 with components of a drug delivery device;
- Fig. 5: a length cut through a drug delivery device according to the present invention with an inventive drive
- Fig. 6: a second embodiment of the drive in a plain view onto a face thereof.

In the figures, the same components are given the same reference symbols.

### Preferred embodiments

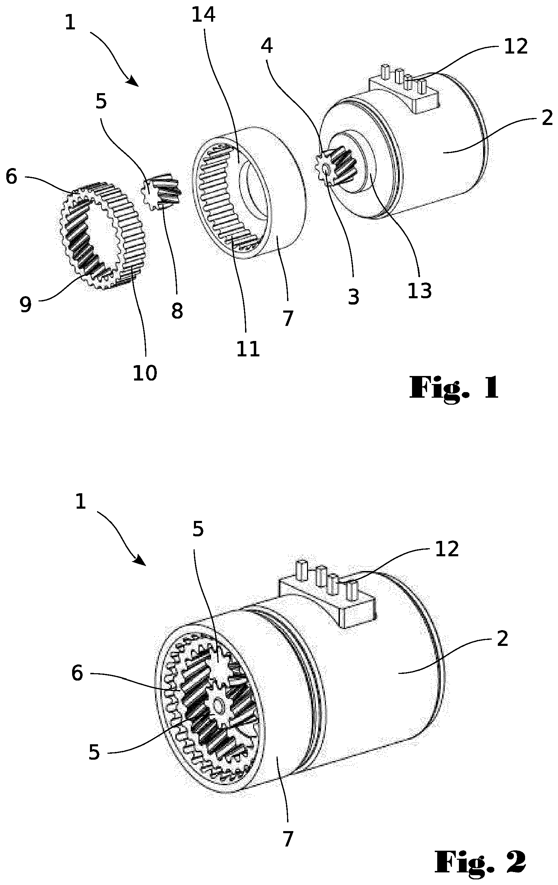

Fig. 1 shows an exploded view of an inventive drive 1 for a drug delivery device. The drive 1 comprises an electric motor 2, preferably a stepper motor. The electric motor 2 has a drive shaft 3 which is driven in rotation. A pinion 4 is arranged on the drive shaft 3. The pinion 4 has helical teeth (inclined teeth) arranged on an outer circumference thereof. In contrast to a straight toothing the helical teeth provide a smooth and quiet run.

The electric motor 2 further includes electrical connectors 12 as well as a shoulder 13 encircling the drive shaft 3.

A stationary ring gear 7 is arranged on a first face of the electric motor 2. The stationary ring gear 7 comprises a plurality of pins 11 on an inner surface thereof which protrude in a radial direction towards the centre of the stationary ring gear 7. The stationary ring gear 7 is shaped as a hollow cylinder with a radial disc 14 which closes parts of a face of the stationary ring gear 7, leaving out a central opening, which is dimensioned such as to be arranged on the shoulder 13 of the electric motor 2. Hence, the stationary ring gear 7 may be precisely arranged on the first face of the electric motor 2.

A cycloidal disc 6 is arranged inside the stationary ring gear 7. The cycloidal disc 6 comprises straight teeth 10 on an outer circumference thereof. The cycloidal disc 6 comprises fewer straight teeth 10 than the number of pins 11 of the stationary ring gear 7. The cycloidal disc 6 is smaller in diameter than the diameter of the inner surface of the stationary ring gear 7. The cycloidal disc 6 may hence be brought into an eccentric movement within the stationary ring gear 7. By this eccentric movement, the straight teeth 10 engage one after the other with the pins 11 of the stationary ring gear 7. As the number of pins 11 is larger than the number of straight teeth 10, each of the straight tooth 10 is slightly offset relative to an opposite pin 11. Hence, when one of the straight teeth 10 engages with an opposite one of the pins 11 the cycloidal disc 6 is rotated around its axis.

In order to impart the eccentric motion to the cycloidal disc 6, a planet gear 5 is arranged between the pinion 4 and the cycloidal disc 6. The cycloidal disc 6 has an internal gear 9 with helical teeth which meshes with helical teeth 8 of the planet gear 5. The planet gear 5 is sized such that the cycloidal disc 6 is pushed into contact with the stationary ring gear 7 at the location opposite where the planet gear 5 meshes with the internal gear 9.

As an example a gear including the pinion 4 and the planet gear 5 each comprising 8 teeth, the cycloidal disc 6 comprising 23 teeth in the inner gear and 26 teeth in the outer gear and the stationary ring gear 7 including 27 teeth results in a gear ratio of 103:1

Fig. 2 shows a perspective view of the drive according to Fig. 1 in an assembled state. As may be seen, the outer diameter of the stationary ring gear 7 corresponds to an outside diameter of the electric motor 2. Hence, the drive 1 forms a compact component which may be inserted into a housing of a drug delivery device. As may also be seen in Fig. 1, the drive 1 according to the present invention comprises only four additional parts besides the electric motor 2.

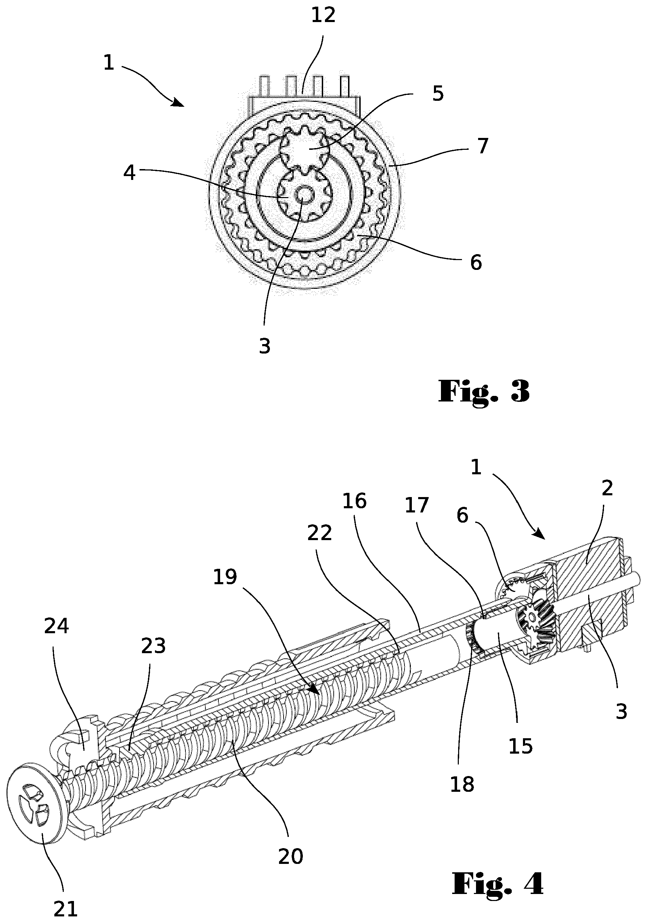

Fig. 3 shows the drive according to Fig. 1 in a plain view onto a face thereof. In this figure, it is well recognizable that the planet gear 5 pushes the cycloidal disc 6 into engagement with the stationary ring gear 7, while the cycloidal disc 6 is totally disengaged from the stationary ring gear 7 at side opposite of the planet gear 5. Further, the gradual offset of the straight teeth 10 of the cycloidal disc 6 relative to the pins **11** of the stationary ring gear 7 may readily be seen.

Fig. 4 is a perspective length section showing the interaction of the drive as shown in Fig. 1 with components of a drug delivery device. The cycloidal disc 6 comprises a protrusion 15 which extends from a first face of the cycloidal disc 6 in an axial direction. The protrusion 15 has the form of a hollow cylinder which is preferably concentric with the cycloidal disc 6. The protrusion 15 comprises outer teeth 17 on an outer surface thereof which mesh with an equal number of inner teeth 18 located on an inner surface of a proximal area of a drive member 16. The diameter of the inner surface of the proximal area of the drive member 16 is chosen such that it equals the diameter of the outermost rotational path of the outer surface of the protrusion 15 which is driven in an eccentric rotational movement, as it is integral with the cycloidal disc 6. By means of this interaction the eccentric rotational movement of the cycloidal disc 6 may be transformed into a centric rotation of the drive member 16.

Alternatively, the drive member 16 may be integral with the cycloidal disc 6. **In** this case a centric bearing bears the distal end of the drive member and the wall thickness and/or material property of the drive member account for a certain radial flexibility at the proximal end of the drive member while transmitting torque from the location of the cycloidal disc 6 to the distal end. Further alternatively, the cycloidal disc 6 or protrusion may engage a threaded rod that in turn drives a hollow plunger rod with an internal thread section at its proximal end.

The drive member 16 is configured as hollow cylinder which is arranged around a plunger rod 19. The plunger rod 19 comprises an outer thread 20. The outer thread 20 meshes with a distal area of the drive member 16. The plunger rod 19 further includes a spline 22 running in an axial direction. A pin which is arranged at a distal end 23 of a holder 24 engages with the spline 22. The pin is part of the holder 24 which is in the shape of a hollow cylinder which encircles the drive member 16. By the engagement of the pin within the spline 22 the plunger rod 19 is prevented from rotating. Hence, when the drive member 16 is driven in rotation, the plunger rod 19 is moved linearly by means of the meshing engagement of the outer thread 20 with the distal area of the drive member 16. At its distal end, the plunger rod 19 comprises a flange 21. The flange 21 pushes on a piston in a reservoir of the delivery device in order to dispense a dose of medicament.

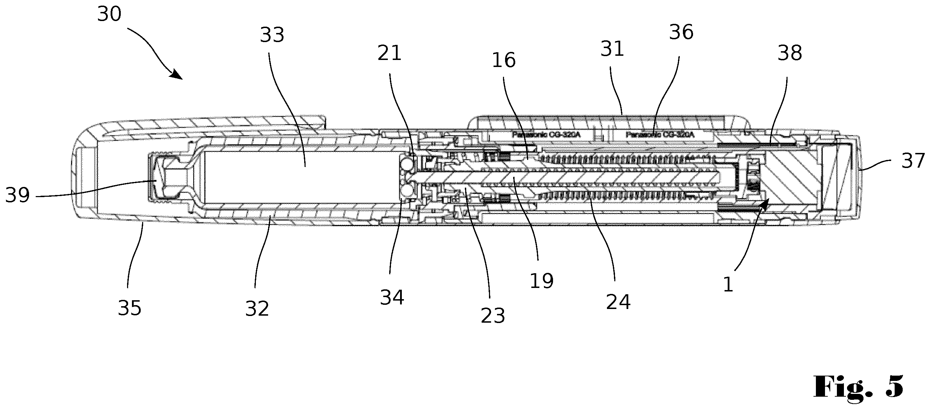

Fig. 5 is a length cut through a drug delivery device 30 according to the present invention with an inventive drive 1. The drug delivery device 30 comprises a longitudinal housing 31 with a circular cross-section. The drive 1 is inserted into the housing 30 from a distal end thereof. Further, a dispensing button 37 is arranged on the distal end of the housing 31. By depressing the dispensing button 37, a defined dose of a medicament is dispensed from the drug delivery device 30. The delivery button 37 as well as the electric motor 2 of the drive are connected to a control element 38. The control element 38 switches the electric motor 2 of the drive 1 on for a pre-set time such that the defined dose of the medicament is dispensed. Batteries 36 are arranged within the housing in order to supply power to the control element 38 and to the electric motor 2 of the drive 1. Further, the holder 24 with the plunger rod 19 are arranged within the housing 31.

A cartridge 32 with a reservoir 33 filled with the medicament is connected to a proximal end of the housing 31. At its distal end, the reservoir 33 comprises a connector 39, in order to connect a needle or cannula to the reservoir 33 for injecting the medicament into tissue of a patient. A piston 34 is arranged within the reservoir 33 which is linearly movable therein in order to displace the medicament for expulsing the medicament through the connector 39. In order to move the piston 34 within the reservoir 33 the piston 34 is in contact with the flange 21 arranged on the distal end of the plunger rod 19. The cartridge 33 comprises a removable cap 39 which encloses the cartridge 33 at its distal end.

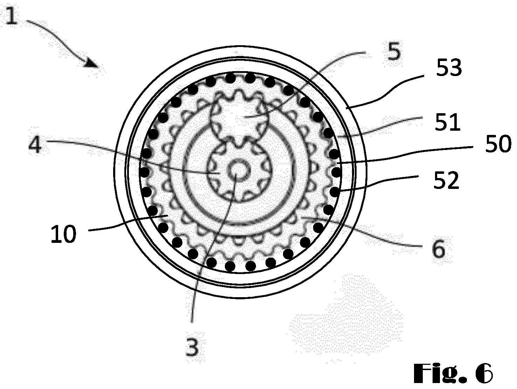

Figure 6 depicts a second embodiment of the drive in a plain view. The drive is similar to the first embodiment and includes a planet gear 5 that pushes a cycloidal disc 6 as described above with respect to figures 1 to 5.

However, in contrast to the first embodiment drive according to the second embodiment as shown in figure 6 comprises a rotationary ring gear 50 including a plurality of cylindrical rollers 52 and an outer rotational sleeve 51.

The rollers 52 are arranged radially outside the cycloidal disc 6 and inside the router rotational sleeve 51. The cycloidal disc 6 is smaller in diameter than an inner diameter of the rotational sleeve 51. The cycloidal disc 6 may hence be brought into the eccentric movement within the rotational sleeve 51. By this eccentric movement, the straight teeth 10 of the cyloidal disc 6 roll over one after the other the rollers 51. As the number of the rollers 52 is larger than the number of straight teeth 10, each of the straight tooth 10 is slightly offset relative to an opposite roller 52. Hence, when one of the straight teeth 10 rolls over an opposite roller 52 the cycloidal disc 6 is rotated around its axis. By this movement the engaged roller 52 is rotated around its rotational axis. As the rollers 52 engage in turn an inner surface of the rotational sleeve 51 the rotated rollers 52 bring the sleeve 51 in rotation. The sleeve 51 is rotationally guided inside a housing 53 and can be connected to a drive member of a drug delivery device, for example as described above with respect to figure 5

Alternatively, the first or the second embodiment of the drive may be implemented in a reusable delivery device including a reset mechanism to reset the piston rod during a replacement of the cartridge or reservoir.

### List of reference symbols

- 1: Drive
- 2: Electric motor
- 3: Drive shaft
- 4: Pinion
- 5: Planet gear
- 6: Cycloidal disc
- 7: Stationary ring gear
- 8: Helical teeth
- 9: Internal gear
- 10: Straight teeth
- 11: Pins
- 12: Electrical connectors
- 13: Shoulder
- 14: Radial disc
- 15: Protrusion
- 16: Drive member
- 17: Outer teeth
- 18: Inner teeth
- 19: Plunger rod
- 20: Outer thread
- 21: Flange
- 22: Spline
- 23: Distal end
- 24: Holder
- 30: Drug delivery device
- 31: Housing
- 32: Cartridge
- 33: Reservoir
- 34: Piston
- 35: Cap
- 36: Battery
- 37: Dispensing button
- 38: Control element
- 39: Connector
- 50: Rotationary ring gear
- 51: Outer rotation sleeve
- 52: Auxiliary roller
- 53: Outer housing

## Claims

1. Drive (1) for a drug delivery device, comprising a drive mechanism with a drive shaft (3), a pinion (4) arranged on the drive shaft (3) and a cycloidal drive, the cycloidal drive having a cycloidal disc (6) and a ring gear (7, 51), **characterized in that** the cycloidal disc (6) is driven in an eccentric motion by means of a planet gear (5), wherein the planet gear (5) is arranged between the cycloidal disc (6) and the pinion (4), wherein the planet gear (5) meshes with the pinion (4) on the drive shaft (3) and with an internal gear of the cycloidal disc (6).

2. Drive (1) according to claim 1, **characterized in that** the drive mechanism is an electric motor (2) and more preferably a stepper motor.

3. Drive (1) according to any of claims 1 or 2, **characterized in that** the pinion (4), the cycloidal disc (6), the ring gear (7) and/or the planet gear (5) are made of a polymer material.

4. Drive (1) according to any of claims 1 to 3, **characterized in that** the planet gear (5) has helical teeth arranged on a peripheral surface.

5. Drive (1) according to any of claims 1 to 4, **characterized in that** the planet gear (5) and the cycloidal drive are configured to provide a reduction gear ratio of at least 50:1, preferably of at least 100:1 between the drive shaft (3) and the cycloidal disc (6).

6. Drive (1) according to any of claims 1 to 5, **characterized in that** the stationary ring gear (7) has an outer diameter which corresponds to or which is smaller than an outer diameter of the drive mechanism.

7. Drive (1) according to any of claims 1 to 6, **characterized in that** the electric motor (2) has a diameter of 10 mm or less.

8. Drive (1) according to any of claims 1 to 7, **characterized in that** the cycloidal disc (6) comprises a cylindrical protrusion which extends from the cycloidal disc in an axial direction which faces away of the drive mechanism.

9. Drive (1) according to any of claims 1 to 8, **characterized in that** the ring gear (50) is rotational and includes an outer rotation sleeve (51) and at least three auxiliary rollers (51) circumferentially arranged on an inside of the rotation sleeve (51) and meshing with an external gear of the cycloidal disc (6).

10. Drug (1) delivery device including a longitudinal housing (31) and a plunger rod (19), the plunger rod (19) being adapted to advance a piston (34) inside a reservoir (33) containing a medicament in order to dispense the medicament, the plunger rod (19) being driven in a dispensing direction by means of the drive according to any of claims 1 to 9 in order to advance the piston (34).

11. Drug delivery device according to claim 10, **characterized in that** the cycloidal disc (6) is rotationally coupled to a drive member (16) which is configured as hollow cylinder being concentrically arranged around the plunger rod (19), the plunger rod (19) having a thread on an outer circumference which meshes with the drive member (16) in a distal area thereof, wherein the plunger rod (19) comprises at least one spline which is engaged with at least one locking pin such as to prevent the plunger rod (19) from rotating.

12. Drug delivery device according to claim 10, **characterized in that** the cycloidal disc (6) is rotationally coupled to a drive member (16) which is configured as hollow cylinder being concentrically arranged around the plunger rod (19), wherein the plunger rod (19) is rotationally coupled to the drive member (16), wherein the plunger rod (19) has a thread on an outer circumference which meshes with the housing (31) or an insert of the housing (31) of the drug delivery device.

13. Drug delivery device according to claim 1 to **11,** wherein a proximal portion of the drive member 16 is rotationally coupled to the cycloidal disc (6) or forms the cyloidal disc (6) and wherein the proximal portion is free to move eccentrically with the cycloidal disc (6) wherein a distal portion of the drive member (16) is rotationally guided.

14. Drug delivery device according to claim 9 to 11, **characterized in** a reset mechanism including a coupling member adapted to selectively couple the plunger rod (19) to the drive member (16) such that a dispensing movement of the drive member (16) is transferred to the plunger rod (19) and to decouple the plunger rod (19) from the drive member (16) such that a movement of the drive member (16) is not transferred to the plunger rod (19) and vice versa.

## Patentansprüche

1. Antrieb (1) für eine Arzneimittelabgabevorrichtung, umfassend einen Antriebsmechanismus mit einer Antriebswelle (3), einem Ritzel (4), das auf der Antriebswelle (3) angeordnet ist, und einem Zykloidgetriebe, wobei das Zykloidgetriebe eine Zykloidscheibe (6) und einen Zahnkranz (7, 51) aufweist, **dadurch gekennzeichnet, dass** die Zykloidscheibe (6) mittels eines Planetenrades (5) in einem exzentrischen Lauf angetrieben wird, wobei das Planetenrad (5) zwischen der Zykloidscheibe (6) und dem Ritzel (4) angeordnet ist, wobei das Planetenrad (5) mit dem Ritzel (4) auf der Antriebswelle (3) und mit einem Innenrad der Zykloidscheibe (6) zusammenpasst.

2. Antrieb (1) nach Anspruch 1, **dadurch gekennzeichnet, dass** der Antriebsmechanismus ein Elektromotor (2) und mehr bevorzugt ein Schrittmotor ist.

3. Antrieb (1) nach einem der Ansprüche 1 oder 2,
**dadurch gekennzeichnet, dass** das Ritzel (4), die Zykloidscheibe (6), der Zahnkranz (7) und/oder das Planetenrad (5) aus einem Polymermaterial hergestellt sind.

4. Antrieb (1) nach einem der Ansprüche 1 bis 3,
**dadurch gekennzeichnet, dass** das Planetenrad (5) eine Schrägverzahnung aufweist, die an einer peripheren Oberfläche angeordnet ist.

5. Antrieb (1) nach einem der Ansprüche 1 bis 4,
**dadurch gekennzeichnet, dass** das Planetenrad (5) und das Zykloidgetriebe konfiguriert sind, um ein Untersetzungsverhältnis von mindestens 50 : 1, vorzugsweise von mindestens 100 : 1, zwischen der Antriebswelle (3) und der Zykloidscheibe (6) bereitzustellen.

6. Antrieb (1) nach einem der Ansprüche 1 bis 5,
**dadurch gekennzeichnet, dass** der stationäre Zahnkranz (7) einen äußeren Durchmesser aufweist, der einem äußeren Durchmesser des Antriebsmechanismus entspricht oder kleiner als dieser ist.

7. Antrieb (1) nach einem der Ansprüche 1 bis 6,
**dadurch gekennzeichnet, dass** der Elektromotor (2) einen Durchmesser von 10 mm oder weniger aufweist.

8. Antrieb (1) nach einem der Ansprüche 1 bis 7,
**dadurch gekennzeichnet, dass** die Zykloidscheibe (6) einen zylindrischen Vorsprung umfasst, der sich von der Zykloidscheibe in einer axialen Richtung erstreckt, die von dem Antriebsmechanismus abgewandt ist.

9. Antrieb (1) nach einem der Ansprüche 1 bis 8,
**dadurch gekennzeichnet, dass** der Zahnkranz (50) rotierend ist und eine äußere Rotationshülse (51) und mindestens drei Hilfsrollen (51) einschließt, die an einer inneren Seite der Rotationshülse (51) umlaufend angeordnet sind und mit einem Außenrad der Zykloidscheibe (6) zusammenpassen.

10. Abgabevorrichtung für ein Arzneimittel (1), die ein longitudinales Gehäuse (31) und eine Druckstange (19) einschließt, wobei die Druckstange (19) angepasst ist, um einen Kolben (34) innerhalb eines Behälters (33) vorzuschieben, der ein Medikament enthält, um das Medikament auszugeben, wobei die Druckstange (19) mittels des Antriebs nach einem der Ansprüche 1 bis 9 in einer Ausgaberichtung angetrieben wird, um den Kolben (34) vorzuschieben.

11. Arzneimittelabgabevorrichtung nach Anspruch 10,
**dadurch gekennzeichnet, dass** die Zykloidscheibe (6) mit einem Antriebselement (16) rotatorisch gekoppelt ist, das als hohler Zylinder konfiguriert ist, der um die Druckstange (19) herum konzentrisch angeordnet ist, wobei die Druckstange (19) an einem äußeren Umfang ein Gewinde aufweist, das mit dem Antriebselement (16) in einem distalen Bereich davon zusammenpasst, wobei die Druckstange (19) mindestens einen Schiebekeil umfasst, der mit mindestens einem Haltestift derart in Eingriff steht, um zu verhindern, dass die Druckstange (19) rotiert.

12. Arzneimittelabgabevorrichtung nach Anspruch 10,
**dadurch gekennzeichnet, dass** die Zykloidscheibe (6) mit einem Antriebselement (16) rotatorisch gekoppelt ist, das als hohler Zylinder konfiguriert ist, der um die Druckstange (19) herum konzentrisch angeordnet ist, wobei die Druckstange (19) mit dem Antriebselement (16) rotatorisch gekoppelt ist, wobei die Druckstange (19) an einem äußeren Umfang ein Gewinde aufweist, das mit dem Gehäuse (31) oder einem Einsatz des Gehäuses (31) der Arzneimittelabgabevorrichtung zusammenpasst.

13. Arzneimittelabgabevorrichtung nach Anspruch 1 bis 11, wobei ein proximaler Abschnitt des Antriebselements 16 mit der Zykloidscheibe (6) rotatorisch gekoppelt ist oder die Zykloidscheibe (6) ausbildet und wobei sich der proximale Abschnitt mit der Zykloidscheibe (6) frei exzentrisch bewegen kann, wobei ein distaler Abschnitt des Antriebselements (16) rotatorisch geführt ist.

14. Arzneimittelabgabevorrichtung nach Anspruch 9 bis 11, **gekennzeichnet durch** einen Rücksetzmechanismus, der ein Kopplungselement einschließt, das angepasst ist, um die Druckstange (19) mit dem Antriebselement (16) derart selektiv zu koppeln, dass eine Ausgabebewegung des Antriebselements (16) auf die Druckstange (19) übertragen wird, und um die Druckstange (19) von dem Antriebselement (16) derart zu entkoppeln, dass eine Bewegung des Antriebselements (16) nicht auf die Druckstange (19) übertragen wird und umgekehrt.

## Revendications

1. Entraînement (1) pour un dispositif d'administration de médicament, comprenant un mécanisme d'entraînement avec un arbre d'entraînement (3), un pignon (4) agencé sur l'arbre d'entraînement (3) et un entraînement cycloïdal, l'entraînement cycloïdal ayant un disque cycloïdal (6) et un engrenage annulaire (7, 51), **caractérisé en ce que** le disque cycloïdal (6) est entraîné dans un mouvement excentrique au moyen d'un engrenage planétaire (5), dans lequel l'engrenage planétaire (5) est agencé entre le disque cycloïdal (6) et le pignon (4), dans lequel l'engrenage planétaire (5) engrène avec le pignon (4) sur l'arbre d'entraînement (3) et avec un engrenage interne du disque cycloïdal (6).

2. Entraînement (1) selon la revendication 1, **caractérisé en ce que** le mécanisme d'entraînement est un moteur électrique (2) et plus préférablement un moteur pas-à-pas.

3. Entraînement (1) selon l'une quelconque des revendications 1 ou 2, **caractérisé en ce que** le pignon (4), le disque cycloïdal (6), l'engrenage annulaire (7) et/ou l'engrenage planétaire (5) sont constitués d'un matériau polymère.

4. Entraînement (1) selon l'une quelconque des revendications 1 à 3, **caractérisé en ce que** l'engrenage planétaire (5) a des dents hélicoïdales agencées sur une surface périphérique.

5. Entraînement (1) selon l'une quelconque des revendications 1 à 4, **caractérisé en ce que** l'engrenage planétaire (5) et l'entraînement cycloïdal sont conçus pour fournir un rapport d'engrenage démultiplicateur d'au moins 50:1, de préférence d'au moins 100:1 entre l'arbre d'entraînement (3) et le disque cycloïdal (6).

6. Entraînement (1) selon l'une quelconque des revendications 1 à 5, **caractérisé en ce que** l'engrenage annulaire (7) fixe a un diamètre externe qui correspond ou qui est inférieur à un diamètre externe du mécanisme d'entraînement.

7. Entraînement (1) selon l'une quelconque des revendications 1 à 6, **caractérisé en ce que** le moteur électrique (2) a un diamètre inférieur ou égal à 10 mm.

8. Entraînement (1) selon l'une quelconque des revendications 1 à 7, **caractérisé en ce que** le disque cycloïdal (6) comprend une saillie cylindrique qui s'étend à partir du disque cycloïdal dans une direction axiale qui fait face à l'opposé du mécanisme d'entraînement.

9. Entraînement (1) selon l'une quelconque des revendications 1 à 8, **caractérisé en ce que** l'engrenage annulaire (50) est rotatif et comporte un manchon de rotation externe (51) et au moins trois rouleaux auxiliaires (51) agencés circonférentiellement sur un intérieur du manchon de rotation (51) et engrenant avec un engrenage externe du disque cycloïdal (6).

10. Dispositif d'administration de médicament (1) comportant un boîtier longitudinal (31) et une tige de piston (19), la tige de piston (19) étant adaptée pour avancer un piston (34) à l'intérieur d'un réservoir (33) contenant un médicament afin de distribuer le médicament, la tige de piston (19) étant entraînée dans une direction de distribution au moyen de l'entraînement selon l'une quelconque des revendications 1 à 9 afin de faire avancer le piston (34).

11. Dispositif d'administration de médicament selon la revendication 10, **caractérisé en ce que** le disque cycloïdal (6) est accouplé en rotation à un élément d'entraînement (16) qui est conçu comme un cylindre creux étant disposé concentriquement autour de la tige de piston (19), la tige de piston (19) ayant un filetage sur une circonférence externe qui s'engrène avec l'élément d'entraînement (16) dans une zone distale de celle-ci, dans lequel la tige de piston (19) comprend au moins une cannelure qui est mise en prise avec au moins une goupille de verrouillage de manière à empêcher la tige de piston (19) de tourner.

12. Dispositif d'administration de médicament selon la revendication 10, **caractérisé en ce que** le disque cycloïdal (6) est accouplé en rotation à un élément d'entraînement (16) qui est conçu comme un cylindre creux étant agencé concentriquement autour de la tige de piston (19), dans lequel la tige de piston (19) est accouplée en rotation à l'élément d'entraînement (16), dans lequel la tige de piston (19) a un filetage sur une circonférence externe qui s'engrène avec le boîtier (31) ou un insert du boîtier (31) du dispositif d'administration de médicament.

13. Dispositif d'administration de médicament selon la revendication 1 à 11, dans lequel une partie proximale de l'élément d'entraînement 16 est accouplée en rotation au disque cycloïdal (6) ou forme le disque cycloïdal (6) et dans lequel la partie proximale est libre de se déplacer de manière excentrique avec le disque cycloïdal (6), dans lequel une partie distale de l'élément d'entraînement (16) est guidée en rotation.

14. Dispositif d'administration de médicament selon la revendication 9 à 11, **caractérisé en ce qu'**un mécanisme de réinitialisation comportant un élément d'accouplement adapté pour accoupler sélectivement la tige de piston (19) à l'élément d'entraînement (16) de sorte qu'un mouvement de distribution de l'élément d'entraînement (16) est transféré à la tige de piston (19) et pour désaccoupler la tige de piston (19) de l'élément d'entraînement (16) de sorte qu'un mouvement de l'élément d'entraînement (16) n'est pas transféré au piston tige (19) et vice versa.
